# EUROPEAN PATENT SPECIFICATION

(11) **EP 2 830 673 B1**
(45) Date of publication and mention of the grant of the patent: **23.06.2021**
(21) Application number: 13714892.0
(22) Date of filing: 27.03.2013
(51) Int. Cl.: A61L 27/30, A61C 13/00, A61C 8/00, A61L 29/10, A61L 31/08

(54) **A MEDICAL DEVICE HAVING A SURFACE COMPRISING GALLIUM OXIDE**
MEDIZINISCHE VORRICHTUNG MIT EINER OBERFLÄCHE MIT GALLIUMOXID
DISPOSITIF MÉDICAL AYANT UNE SURFACE COMPRENANT DE L'OXYDE DE GALLIUM

(30) Priority: 30.03.2012 US 201261617940 P; 30.03.2012 EP 12162632
(43) Date of publication of application: 04.02.2015
(73) Proprietor: Dentsply IH AB, 431 21 Mölndal (SE)
(72) Inventor: ARVIDSSON, Anna, S-412 74 Göteborg (SE); JOHANSSON, Anders, S-756 43 Uppsala (SE); ROOTH, Mårten, S-741 94 Knivsta (SE)
(74) Representative: AWA Sweden AB
(86) International application number: PCT/EP2013/056480
(87) International publication number: WO 2013/144185

(56) References cited:
- WO-A1-2006/004297
- WO-A1-2008/036787
- WO-A2-2007/053581
- WO-A2-2010/025721
- WO-A2-2011/012213
- US-A1- 2006 018 945
- US-A1- 2011 017 659
- US-A1- 2012 059 455
- Viviana Mourino et al.: Advanced Biomaterials, vol. 12, no. 7 30 July 2010 (2010-07-30), pages B283-B291, XP002682583, Weinheim Retrieved from the Internet: URL:http://onlinelibrary.wiley.com/doi/10. 1002/adem.200980078/abstract#fn1 [retrieved on 2012-08-30]
- DATABASE WPI Week 201046 Thomson Scientific, London, GB; AN 2010-H38889 XP002682628, & CN 101 721 742 A (BEIJING NON-FERROUS METAL INST) 9 June 2010 (2010-06-09)
- None

## Description

### Field of the invention

The present invention relates to a medical device having a surface layer comprising gallium oxide, and to methods of producing such a device.

### Background of the invention

For any type of medical device intended for contact with living tissue, biocompatibility is a crucial issue. The risk for foreign body reaction, clot formation and infection, among many other things, must be addressed and minimized in order to avoid adverse effects, local as well as systemic, which may otherwise compromise the health of the patient and/or lead to failure of the device. This is particularly the case for permanent implants.

Healing or regeneration of tissue around an implant is often vital in order to secure the implant and its long-term functionality. This is especially important for load-bearing implants such as dental or orthopedic implants.

Dental implant systems are widely used for replacing damaged or lost natural teeth. In such implant systems, a dental fixture (screw), usually made of titanium or a titanium alloy, is placed in the jawbone of the patient in order to replace the natural tooth root. An abutment structure is then attached to the fixture in order to build up a core for the part of the prosthetic tooth protruding from the bone tissue, through the soft gingival tissue and into the mouth of the patient. On said abutment, the prosthesis or crown may finally be seated.

For dental fixtures, a strong attachment between the bone tissue and the implant is necessary. For implants intended for contact with soft tissue, such as abutments which are to be partially located in the soft gingival tissue, also the compatibility with soft tissue is vital for total implant functionality. Typically, after implantation of a dental implant system, an abutment is partially or completely surrounded by gingival tissue. It is desirable that the gingival tissue should heal quickly and firmly around the implant, both for medical and aesthetic reasons. A tight sealing between the oral mucosa and the dental implant serves as a barrier against the oral microbial environment and is crucial for implant success. This is especially important for patients with poor oral hygiene and/or inadequate bone or mucosal quality. Poor healing or poor attachment between the soft tissue and the implant increases the risk for infection and peri-implantitis, which may ultimately lead to bone resorption and failure of the implant.

There are several strategies for increasing the chances of a successful implantation of a medical device, for example enhancing the rate of new tissue formation and/or, in instances where tissue-implant bonding is desired, enhancing the rate of tissue attachment to the implant surface, or by reducing the risk for infection. Enhancement of new tissue formation may be achieved for example by various surface modifications and/or deposition of bioactive agents on the surface.

The risk of infection in connection with dental implants is today primarily addressed by preventive measures, such as maintaining good oral hygiene. Once a biofilm is formed on the surface of a dental implant, it is difficult to remove it by applying antibacterial agents. In the case of infection in the bone or soft tissue surrounding a dental implant (peri-implantitis), mechanical debridement is the basic element, sometimes in combination with antibiotics, antiseptics, and/or ultrasonic or laser treatment. WO 2007/053581 A2 discloses to apply gallium compounds containing coatings to disinfect or protect the surfaces from microbial contaminations.

### Summary of the invention

It is an object of the present invention to overcome this problem, and to provide a medical device as defined in the appended claims.

According to a first aspect of the invention, this and other objects are achieved by medical device intended for contact with living tissue, comprising a substrate having a surface layer comprising gallium oxide, in particular Ga₂O₃. The layer comprising has an atomic concentration (at%) of gallium of at least 5 at%. In embodiments of the invention, the gallium concentration in said layer is at least 10 at%, more preferably at least 15 at%, and even more preferably at least 20 at%. The layer may have a gallium content of up to 40 at%.

A medical device surface having a layer incorporating gallium oxide has been shown to be effective against various bacterial strains, and was shown to inhibit biofilm formation in vitro. The medical device according to the invention may also be effective against other microbes, such as fungi.

In embodiments of the invention said living tissue is soft tissue. Alternatively, said living tissue may be cartilage or bone tissue.

Gallium oxide is in general well tolerated by living tissue of a mammal, and can be deposited on a surface using a thin film deposition technique. Gallium oxide is useful in the present invention, in particular for dental implant applications, because it may provide an aesthetically desirable surface layer, in particular with respect to color. Gallium oxides such as Ga₂O₃ can be deposited using thin film deposition techniques, including atomic layer deposition.

In embodiments of the invention, the layer comprising a gallium compound further comprises a gallium salt. For example a gallium salt may be deposited onto a first layer comprising a first gallium compound, e.g. gallium oxide. A gallium salt deposit may increase the release of gallium from the surface at early after contact with living tissue, thus temporarily further enhancing an antibacterial or antimicrobial effect of the layer.

Generally, the layer comprising the gallium compound has a thickness in the range of from 10 nm to 1.5 µm, preferably from 10 nm to 1 µm, such as from 10 nm to 100 nm. A layer of at least 10 nm is sufficient to provide a desirable antibacterial effect, whereas thick layers of up to 1 µm may be desirable for aesthetic reasons, having a color suitable for e.g. dental implants.

In embodiments of the invention, the gallium oxide may be crystalline. In other embodiments, the gallium oxide may be amorphous.

Typically, in embodiments of the invention, the layer comprising the gallium oxide may be a homogeneous layer. The layer is a non-porous layer. A non-porous layer is typically less susceptible of bacterial growth and biofilm formation compared to a porous layer.

The substrate on which the layer comprising the at least one gallium compound is provided may comprise a metallic material, preferably titanium or titanium alloy. Alternatively, the substrate may comprise a ceramic material. In other embodiments, the substrate may comprise a polymeric material, or a composite material.

The medical device of the invention is typically an implant intended for long-term contact with, or implantation into, living tissue. In one embodiment the medical device is an implant intended for implantation at least partially into soft tissue. For example, the medical device may be a dental implant, in particular a dental abutment. In another embodiment, the medical device may be a bone anchored hearing device. In yet other embodiments of the invention, the medical device may be intended for short-term or prolonged contact with living tissue, typically soft tissue. For example, the medical device may be a catheter adapted for insertion into a bodily cavity such as a blood vessel, the digestive tract or the urinary system.

In another aspect, the invention provides a method of producing a medical device as described in the claims, comprising
a) providing a substrate having a surface; and
b) applying gallium oxide onto said surface to form a layer.

Applying the Ga₂O₃ can be achieved using a thin film deposition technique, for example atomic layer deposition.

A medical device as described above may be used for preventing biofilm formation and/or bacterial infection of a surrounding tissue, in particular soft tissue. In particular the medical device of the invention may be used for preventing bacterial infection of gingival tissue and/or periimplantitis.

It is noted that the invention relates to all possible combinations of features recited in the claims.

### Brief description of the drawings

Figure 1 is a side view of a medical device according to an embodiment of the invention, wherein the medical device is a dental abutment.
Figure 2 illustrates in cross-section part of a medical device according to embodiments of the invention, showing a substrate material and a layer comprising gallium oxide.

### Detailed description of the invention

It has been found that a medical device having a surface layer comprising a gallium oxide, notably Ga₂O₃, provides very advantageous effects in terms of reduced risk of infection, improved tissue healing and/or aesthetic performance. It has been demonstrated that a titanium body having a surface incorporating gallium (Ga) in the form of a coating of gallium oxide (Ga₂O₃) can prevent the growth of bacteria on and around the surface and thus may be useful in preventing detrimental infection around e.g. a dental abutment implanted into the gingiva.

According to the present invention, a tissue contact surface of a surface of a medical device comprises gallium oxide in the form of Ga₂O₃. For example, the gallium oxide may be applied to a medical device as a surface layer.

Gallium has been used in medicine at least since the 1940's, primarily as a radioactive agent for medical imaging. The antibacterial properties of gallium have been investigated in several studies. In Kaneko et al. (2007) it was established that gallium nitrate (Ga(NO₃)₃) inhibits growth of *Pseudomonas aeruginosa* in batch cultures. Olakanmi et al (2010) found that Ga(NO₃)₃ inhibited the growth of *Francisella novicida.* Gallium acts by disrupting iron metabolism. It may be assumed that gallium is also effective against other microbes, e.g. fungi such as yeasts or moulds.

Directive 2007/47/ec defines a medical device as: "any instrument, apparatus, appliance, software, material or other article, whether used alone or in combination, including the software intended by its manufacturer to be used specifically for diagnostic and/or therapeutic purposes and necessary for its proper application, intended by the manufacturer to be used for human beings". In the context of the present invention, only medical devices intended for contact with living tissue are considered, that is, any instrument, apparatus appliance, material or other article of physical character that is intended to be applied on, inserted into, implanted in or otherwise brought into contact with the body, a body part or an organ. Furthermore, said body, body part or organ may be that of a human or animal, typically mammal, subject. Preferably however the medical device is intended for human subjects. Medical devices included within the above definition are for example implants, catheters, shunts, tubes, stents, intrauterine devices, and prostheses.

In particular, the medical device may be a medical device intended for implantation into living tissue or for insertion into the body or a body part of a subject, including insertion into a bodily cavity.

The present medical device may be intended for short-term, prolonged or long-term contact with living tissue. By "short-term" is meant a duration of less than 24 hours, in accordance with definitions found in ISO 10993-1 for the biological evaluation of medical devices. Furthermore, "prolonged", according to the same standard, refers to a duration of from 24 hours up to 30 days. Accordingly, by the same standard, by "long-term" is meant a duration of more than 30 days. Thus, in some embodiments the medical device of the invention may be a permanent implant, intended to remain for months, years, or even life-long in the body of a subject.

As used herein the term "implant" includes within its scope any device of which at least a part is intended to be implanted into the body of a vertebrate animal, in particular a mammal, such as a human. Implants may be used to replace anatomy and/or restore any function of the body. Generally, an implant is composed of one or several implant parts. For instance, a dental implant usually comprises a dental fixture coupled to secondary implant parts, such as an abutment and/or a restoration tooth. However, any device, such as a dental fixture, intended for implantation may alone be referred to as an implant even if other parts are to be connected thereto.

By "biocompatible" is meant a material which, upon contact with living tissue, does not as such elicit an adverse biological response (for example inflammation or other immunological reactions) of said tissue.

By "soft tissue" is meant any tissue type, in particular mammalian tissue types, that is not bone or cartilage. Examples of soft tissue for which the medical device is suitable include, but are not limited to, connective tissue, fibrous tissue, epithelial tissue, vascular tissue, muscular tissue, mucosa, gingiva, and skin.

As used herein, "homogeneous layer" refers to a layer having a chemical composition that is uniform in all directions (three dimensions).

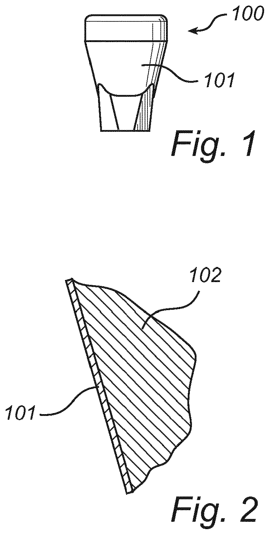

Figures 1 and 2 illustrate an embodiment according to the present invention in which the medical device is a dental abutment. The dental abutment 100 comprises a body of substrate material 102 coated with a layer 101 comprising gallium oxide. The layer 101 forms the surface of the abutment intended to face and contact the gingival tissue after implantation.

The medical device of the invention may be made of any suitable biocompatible material, e.g. materials used for implantable devices. Typically the medical device comprises a substrate having a surface which comprises a gallium compound. The substrate may for example be made of a biocompatible metal or metal alloy, including one or more materials selected from the group consisting of titanium, zirconium, hafnium, vanadium, niobium, tantalum, cobalt and iridium, and alloys thereof. Alternatively, the substrate of the medical device may be made of a biocompatible ceramic, such as zirconia, titania, shape memory metal ceramics and combinations thereof. In embodiments where the medical device is used as or forms part of a dental abutment, the substrate is preferably made of a metallic material.

In contact with oxygen, the metals titanium, zirconium, hafnium, tantalum, niobium and their alloys instantaneously react to form an inert oxide. Thus, the surfaces of articles of these materials are virtually always covered with a thin oxide layer. The native oxide layer of a titanium substrate mainly consists of titanium(IV) dioxide (TiO₂) with minor amounts of Ti₂O₃, TiO and Ti₃O₄.

Thus, in embodiments where the medical device comprises one or more of titanium, zirconium, hafnium, tantalum, niobium or an alloy of any one thereof, the medical device typically has a native metal oxide surface layer. Such a native metal oxide layer may, in turn, be covered by a thin film comprising Ga₂O₃.

In other embodiments of the present invention, the medical device, in particular the substrate, may be made of a biocompatible polymer, typically selected from the group consisting of polyether ether ketone (PEEK), poly methyl methacrylate (PMMA), poly lactic acid (PLLA) and polyglycolic acid (PGA) and any combinations and copolymers thereof.

In embodiments of the invention, the medical device is intended for short-term, prolonged or long-term contact with living tissue. For example, the medical device of the invention may be an implant, typically intended to temporarily or permanently replace or restore a function or structure of the body.

Typically, at least part of the surface of the medical device is intended for contact with soft tissue, and at least part of this soft tissue contact surface has a layer comprising Ga₂O₃. For example, the medical device may be an implant intended for contact primarily or exclusively with soft tissue, for example a dental abutment. Alternatively, the medical device may be an implant to be inserted partially in bone and partially in soft tissue. Examples of such implants include one-piece dental implants and bone-anchored hearing devices (also referred to as bone anchored hearing aids). Where only part of the implant is intended for contact with soft tissue, it is preferred that the layer comprising the gallium oxide is provided at least on a part of a soft tissue contact surface.

The medical device may also be suitable for contact with cartilage.

In other embodiments, the medical device may be intended for contact with bone tissue, e.g. the jawbone, the femur or the skull of a mammal, in particular a human. Examples of such medical devices include dental fixtures and orthopedic implants.

According to the present invention, the surface layer comprises gallium oxide (Ga₂O₃). Gallium oxide may be present in amorphous or crystalline form. Crystalline forms of gallium oxide include α-Ga₂O₃, β-Ga₂O₃, γ-Ga₂O₃, δ-Ga₂O₃, and ε-Ga₂O₃. Furthermore, a gallium oxide surface layer may be at least partially hydroxylated to form hydroxy oxide.

Not wishing to be bound by any particular theory, it is believed that upon contact with living tissue and/or body fluids, a layer of gallium oxide exhibits slow, sustained release of gallium ions. Such release may be slower and more sustained compared to the release of gallium ions from a precipitated gallium salt, and may thus provide a more long-term effect with respect to biofilm formation. In addition, a surface layer deposited using a thin film deposition method as used in embodiments of the invention may adhere firmly to the underlying substrate and thus may avoid problem related to peeling and flaking of the surface layer. Peeling and flaking may give rise to adverse inflammatory response of the surrounding tissue, and in addition may undermine the biofilm prevention effect of the surface layer.

Depending on the intended use of the medical device, different release properties may be desirable. For example, a higher release rate of gallium may be more favorable for short term use, i.e. for a medical device intended for short-term contact with living tissue, compared to a device intended for prolonged or long-term contact. The release rate may be affected by various factors, for example the crystallinity of the gallium oxide. Optionally, in embodiments of the invention the medical device may additionally comprise a gallium salt selected from the group consisting of gallium acetate, gallium carbonate, gallium chloride, gallium citrate, gallium fluoride, gallium formate, gallium iodide, gallium lactate, gallium maltolate, gallium nitrate, gallium oxalate, gallium phosphate, and gallium sulphate. Such a gallium salt may be provided as a deposit, e.g. precipitated, on the layer comprising the gallium compound.

As mentioned above, the gallium oxide is typically contained in an applied surface layer. In embodiments of the invention, the gallium oxide may constitute the major part of said layer. The atomic concentration (at%) of the elements together forming the gallium oxide constitute at least 50 at% of the layer, preferably at least 70 at% and more preferably at least 80 at% of the elements of the layer. The atomic concentration of gallium in the layer is from 5 at% and may be up to 40 at%, for example at least 10 at%, at least 15 at%, at least 20 at%, at least 30 at% or at least 35 at%, and up to 40 at%.

Using a layer comprising gallium oxide (Ga₂O₃), the maximum content of gallium in the layer is 40 at%, and the maximum content of oxygen in the layer is 60 at%. However, impurities and contamination, for example carbon, may be present at up to 20 at%.

The atomic concentration may be measured for example to a depth of 40 nm or less, and preferably not more than the layer thickness. The atomic concentration can be measured using X-ray photoelectron spectroscopy (XPS).

As mentioned above, upon contact with living tissue, some gallium may be released from the surface of the medical device over time. Hence after implantation the content of gallium and possibly also of other materials present on the surface of the medical device may change over time.

Furthermore, the layer comprising the gallium oxide may contain impurities or contamination, for example carbon, typically in an amount of 20 at% or less, and preferably 15 at% or less, or 10 at% or less. Such contamination may originate e.g. from the packaging. It may be noted that wet packaging, in which the surface may be protected by water, ethanol or the like, reduces the amount of contamination by carbon, compared to dry packaging where the surface is exposed to air which normally contains volatile hydrocarbons. Contamination may also present on the surface of the substrate before the layer comprising the gallium oxide is applied. The level of contamination, typically represented by the atomic concentration of carbon, may be reduced by cleaning the surface before applying the gallium oxide, and optionally after applying the gallium oxide and/or by avoiding further contaminating the surface before measuring the atomic concentration of elements on the surface.

The maximum atomic concentration of the elements of the gallium oxide in the layer can easily be determined from the composition stoichiometry.

Table 1 summarizes possible atomic concentration ranges for a layer comprising gallium oxide.

**Table 1. Exemplary atomic concentrations of the elements of gallium oxide.**

| | **Atomic concentration (at%)** |
|---|---|
| **Ga** | 5-40 at% |
| **O** | 7.5-60 at% |

In some embodiments, the surface layer consists essentially of gallium oxide. In accordance with the above, "consists essentially of" here means that the layer contains little or no other material (contaminants, etc) except the gallium oxide, only for example up to 10 at%, preferably up to 5 at%, more preferably up to 2 at% and even more preferably up to 1 at% of other material.

In general, the layer comprising the gallium oxide is free of carrier material such as polymers, solvents, etc.

The layer comprising the gallium oxide has a thickness in the range of from 10 nm to 1.5 µm. A layer having a thickness of at least 1 nm may provide sufficient antimicrobial effect. Increasing layer thickness may provide a whiter color, which may be desirable for dental applications. However, also a layer having a thickness of from about 10 nm may be more aesthetically advantageous than present commercial dental abutments. For example, a gallium oxide layer of 40 nm has a deep bronze color which would be less visible through a patient's gingiva than current grey-metallic titanium abutments.

Where mainly an antimicrobial effect is sought, the layer containing the gallium oxide may have a thickness of from 10 to 100 nm, or optionally up to 300 nm. On the other hand, where the aesthetic appearance of e.g. a dental abutment is of high importance, a layer thickness in the range of from 0.5 to 1.5 µm, e.g. from 0.7 to 1.5 µm or from 0.7 to 1 µm may be preferred. However also thinner layers may provide an acceptable color appearance and which at least may be more advantageous than prior art dental abutments.

The layer comprising the gallium oxide is a dense layer, i.e. a non-porous layer.

In embodiments of the invention, the surface of the medical device may comprise a single layer. Alternatively, in other embodiments, the medical device may comprise multiple layers, at least one comprising the gallium oxide.

In embodiments of the invention, a gallium salt, optionally forming a further layer, may be provided on at least a portion of a thin-film deposited layer comprising a gallium compound. For example, a solution of at least one gallium salt may be applied onto a thin-film deposited layer of the gallium compound, and allowed to evaporate. Such embodiments may provide a high initial release of gallium upon contact with living tissue, which may be advantageous in many instances, for short-term, prolonged as well as for long-term tissue contact.

In embodiments of the invention, the substrate may have a rough surface on which a layer comprising the gallium oxide is arranged. Since the layer comprising the gallium oxide may be thin, e.g. 100 nm or less, it may have good conformal step coverage, meaning that the layer comprising the gallium oxide follows the underlying surface roughness and substantially preserves it, without making it smoother. However, in embodiments where the layer comprising the gallium oxide is relatively thick, it may reduce the roughness of the underlying substrate surface.

The substrate surface roughness, and hence optionally also the surface of the medical device formed by the layer comprising the gallium oxide, may have an average surface roughness Rₐ of at least 0.05 µm, typically at least 0.1 µm, for example at least 0.2 µm. Since surfaces having an average surface roughness (Rₐ) of at least 0.2 µm are believed to be more susceptible of biofilm formation, a layer comprising gallium oxide as described herein may be particularly advantageous for medical devices having a surface roughness of at least 0.2 µm, and may be increasingly useful for preventing biofilm formation on medical devices having even higher surface roughness. As an example, a dental abutment comprising a titanium substrate may have a surface roughness of about 0.2-0.3 µm. A surface layer of gallium oxide having a thickness of about 40 nm may substantially preserve this surface roughness (which may be desirable e.g. in order to facilitate a firm anchorage of the implant in the surrounding tissue) but may prevent biofilm formation on the implant surface and hence reduce the risk for infection and periimplantitis.

The layer comprising gallium oxide may be formed by applying the gallium oxide onto the surface of a medical device, to form a surface layer. The gallium oxide may be applied using known deposition techniques, especially thin film deposition techniques. Suitable techniques may include physical deposition, chemical deposition and physical-chemical deposition. One example of such techniques is atomic layer deposition (ALD) which can be used to provide e.g. a gallium oxide layer on a substrate surface (Nieminen et al, 1996; Shan et al, 2005).

ALD and other thin film deposition techniques are associated with several advantages for the deposition of the gallium compound(s), such as controlled layer thickness, controlled composition, high purity, conformal step coverage, good uniformity (resulting in a homogeneous layer), and good adhesion.

### Examples

### Example 1. Production

Coins of commercially pure (cp) titanium (grade 4) were manufactured and cleaned before deposition of a 40 nm thick layer of amorphous Ga₂O₃ using atomic layer deposition (Picosun, Finland) with precursors of GaCl₃ and H₂O, respectively. Specimens were thereafter packaged in plastic containers, and sterilized with electron beam irradiation.

### Example 2. Surface characterization

For all surface characterization experiments, eight specimens each of commercially pure (cp) titanium, Ga₂O₃ coated cp titanium produced as described above, and commercially available TiN coated cp titanium, were prepared as described in Example 1 (cleaned, coating using ALD in the case of the Ga₂O₃ coated specimens, packaged, and sterilized). The TiN coated specimens were included for comparison since it is known that a TiN coating provides a weakly antibacterial effect.

It was found that the surface morphology and surface roughness was unaltered by the ALD coating, but there was a slight increase of hydrophobic properties.

### a) Surface chemistry

Surface morphology and surface chemistry was analyzed with environmental scanning electron microscopy (XL30 ESEM, Philips, Netherlands)/energy dispersive spectroscopy (Genesis System, EDAX Inc., USA) at an acceleration voltage at 10-30 kV. Elements detected on the surface of the Ga₂O₃ coated specimens were oxygen (O), gallium (Ga), and titanium (Ti). Ga concentrations varied between 4 to 9 atomic % (at%), as measured with acceleration voltages at 30 kV and 10 kV, respectively. The analytical depth with this technique is estimated to be approximately 1 µm, i.e. much deeper than the layer thickness. No differences in terms of surface morphology could be detected between commercially pure titanium controls and the Ga₂O₃ coated titanium.

Additionally, surface chemistry was analyzed with X-ray photoelectron spectroscopy (XPS, Physical Electronics, USA), which is a more surface sensitive technique than energy dispersive spectroscopy. As X-ray source monochromatic AIKα was used. The beam was focused to 100 µm. Elements detected were oxygen (O), gallium (Ga), and carbon (C). Gallium concentrations varied between 34 and 37 at%. Oxygen concentrations varied between 47 and 50 at%. The analytical depth with this technique is estimated to be approximately 5-10 nm. The results are summarized in Table 2.

**Table 2. Atomic concentration of detected elements using XPS**

| **Element** | **At% detected using XPS (depth 5-10 nm)** |
|---|---|
| Ga | 34-37 at% |
| O | 47-50 at% |
| C | 13-18 at% |

### b) Surface morphology

Surface roughness was measured with surface profilometry (Hommel T1000 wave, Hommelwerke GmbH, Germany). A vertical measuring range of 320 µm, and an assessment length of 4.8 mm were used. Two specimens of each type were included in the analysis, and three measurements per specimen were performed. The surface roughness Rₐ was calculated after using a filtering process, with cut-off at 0.800 mm. The results are presented in Table 3.

**Table 3. Surface roughness (Rₐ) ± standard deviations (SD)**

| **Test specimens** | **Rₐ (µm)** |
|---|---|
| TiN coated titanium | 0.28 ± 0.01 |
| Ga₂O₃ coated titanium | 0.31 ± 0.04 |
| Uncoated titanium | 0.34 ± 0.03 |

### c) Wettability

In order to investigate the wettability, the contact angle was measured using a contact angle measuring system (Drop Shape Analysis System DSA 100, Kruss GmbH, Germany). Measurements were performed with deionized water. The results indicate that all specimens were hydrophobic (>90°), see Table 4.

**Table 4. Contact angles (°) ± standard deviations (SD)**

| **Test specimens** | **Contact angle (°)** |
|---|---|
| TiN coated titanium | 95.0 ± 1.4 |
| Ga₂O₃ coated titanium | 97.7 ± 2.2 |
| Uncoated titanium | 92.0 ± 2.8 |

### Example 3. Antimicrobial effect of gallium oxide-coated surfaces

It was found that a titanium body having a surface comprising gallium (Ga) in the form gallium oxide can prevent the growth of *Pseudomonas aeruginosa* and *Staphylococcus aureus* on and around a surface and thus may be useful in preventing detrimental infection around e.g. a dental abutment implanted into the gingiva.

### a) Inhibition of bacterial growth on streak plate

In a first experiment commercially pure titanium coins (∅ 6,25 mm) with or without a gallium oxide coating were placed on agar plates containing homogeneously distributed colonies of *Pseudomonas aeruginosa.* After incubation for 24 hours at 37°C there was a 4 mm wide visible colony free zone surrounding the gallium oxide coins, in contrast to the titanium coins that were surrounded by bacterial colonies.

### b) Inhibition of bacterial growth using film contact method

In a second experiment, a film contact method (Yasuyuki et al, 2010) was used. Streak plates of *Pseudomonas aeruginosa* (PA01) or methillicin resistant *Staphylococcus aureus* (MRSA) were made and 1 colony was inoculated to 5 ml tryptic soy broth (TSB) in culture tubes and grown under shaking conditions for 18 hours. Cell density was measured in a spectrophotometer at OD 600 nm and counted using a cell counting chamber. The cell culture was adjusted with sterile TSB to 1-5 X 10⁶ cells/ml. Specimens of commercially pure (cp) titanium coins (∅ 6.25 mm), cp titanium coins with a gallium oxide coating, or cp titanium coins with a commercially available titanium nitride (TiN) coating were aseptically prepared and put in respective well of a 12 well plate. Thin transparent plastic film was punched, and sterilized using 70 % ethanol and UV irradiation on each side. A 15µl drop of bacteria in TSB was applied on each specimen. One thin plastic film per specimen was placed over the bacteria on the specimens so that the bacterial solution was evenly spread over the specimen surface, ensuring good contact. After incubation for 24 hours at 30±1 °C, the film of each specimen was aseptically removed and washed by pipetting 1ml PBS over the surface into a separate 2 ml eppendorf tube per specimen. The specimens were transferred to the same eppendorf tubes as used when washing the film. First each specimen surface was washed by pipetting the very same PBS as the film was previously washed with. Next, the specimens were sonicated and for 1 minute and vigorously vortexed for 1 minute in the very same tube as previously used when washing the film. Serial dilutions and plate count were performed. Plates were incubated for 24 hours and colony numbers counted and recorded. The antibacterial activity of gallium oxide coated titanium was determined to 92 % reduction against PA01 and 71 % reduction against MRSA, compared to titanium, see Tables 5 and 6.

**Table 5. Viable counts (cfu/ml) ± standard deviations (SD) after 24 hours incubation of test specimens aginst Pseudomonas Aeruginosa (PA01).**

| **Test specimens** | **PA01 (cfu/ml±SD) 24h** |
|---|---|
| TiN coated titanium | 1.6E+08 ± 1.2E+08 |
| Ga₂O₃ coated titanium | 7.4E+07 ± 2.8E+06 |
| Uncoated titanium | 1.0E+09 ± 6.0E+08 |

**Table 6. Viable counts (cfu/ml) ± standard deviations (SD) after 24 hours aginst methillicin resistant Staphylococcus aureus (MRSA).**

| **Test specimens** | **MRSA (cfu/ml±SD) 24h** |
|---|---|
| TiN coated titanium | 5.0E+08 ± 6.0E+07 |
| Ga₂O₃ coated titanium | 2.2E+08 ± 3.7E+07 |
| Uncoated titanium | 7.6E+08 ± 6.8E+07 |

### c) In situ effect on a biofilm

In a third experiment, the antibacterial activity of titanium discs, with or without a gallium oxide coating, was evaluated *in situ* using Live/Dead® BacLight™ stain (Life Technologies Ltd, UK). Streak plates of *Pseudomonas aeruginosa* (PA01) were made and 1 colony was inoculated to 5 ml TSB in culture tubes and grown under shaking conditions for 18 hours. Cell density was measured in a spectrophotometer at OD 600 nm and adjusted with sterile TSB to 1 x 10⁶ cells/ml. 400 µl bacteria were aliquoted into 8-chambered slides. The biofilm was allowed to be formed during 24 hours at 35±2°C. Specimens of commercially pure (cp) titanium coins (∅ 6.25 mm), cp titanium coins with a gallium oxide coating, or cp titanium coins with a titanium nitride (TiN) coating were aseptically prepared and applied onto the biofilm. The antibacterial activity was analyzed in situ using the Live/Dead® stain.

In situ analyses indicated that both titanium nitride and gallium oxide coatings have an anti-biofilm activity compared with uncoated titanium in terms of viability. At 24 hour analysis, it was visualized that the typical mushroom structure of biofilms had disappeared for titanium nitride and gallium oxide. It was also found that more dead cells were seen on gallium oxide than on titanium nitride.

The person skilled in the art realizes that the present invention by no means is limited to the preferred embodiments described above. On the contrary, many modifications and variations are possible within the scope of the appended claims.

Additionally, variations to the disclosed embodiments can be understood and effected by the skilled person in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

### References

1. Y. Kaneko, M. Thoendel, O. Olakanmi, B. E. Britigan and P. K. Singh, The Journal of Clinical Investigation, Vol 117 (2007) 877-888.
2. M. Nieminen, L. Niinistö and E. Rauhala. J Mater Chem 6 (1996) 27-31.
3. O. Olakanmi J. S. Gunn, S. Su, S. Soni, D. J. Hassett, B. E. Britigan. Antimicrobial agents and Chemotherapy 54 (2010) 244-253.
4. F. K. Shan, G. X. Liu, W. J. Lee, G. H. Lee, I. S. Kim et al. J Appl Physics 98 (2005) 023504-1-6.
5. M. Yasuyuki, K. Kunihiro, S. Kurissery, N. Kanavillil, Y. Sato, Y. Kikuchi. Biofouling 26 (2010) 851-858.

## Claims

1. A medical device intended for contact with living tissue, said medical device being selected from the group consisting of a dental implant, a bone anchored hearing device and an orthopaedic implant and comprising a substrate having an antimicrobial surface layer comprising Ga₂O₃, **characterized in that** said layer is non-porous and has a thickness in the range of from 10 nm to 1.5 µm, and has a gallium content of at least 5 at%.

2. The medical device according to claim 1, wherein said living tissue is soft tissue.

3. The medical device according to any one of the preceding claims, wherein said layer has a thickness in the range of from 10 nm to 1 µm.

4. The medical device according to any one of the preceding claims, wherein said layer has a gallium content of at least 10 at%, preferably at least 20 at%.

5. The medical device according to any one of the preceding claims, wherein said layer has a gallium content of up to 40 at%.

6. The medical device according to any one of the preceding claims, wherein said layer is a homogeneous layer.

7. The medical device according to any one of the preceding claims, wherein said substrate comprises a metallic material, preferably titanium or titanium alloy.

8. The medical device according to any one of the claims 1 to 6, wherein said substrate comprises a ceramic material.

9. The medical device according to any one of the claims 1 to 6, wherein said substrate comprises a composite material.

10. The medical device according to any one of the preceding claims, wherein said implant is a dental implant.

11. The medical device according to claim 10, wherein said dental implant is a dental abutment.

12. A method of producing a medical device according to any one of the claims 1 to 11 comprising
a) providing a substrate having a surface; and
b) applying Ga₂O₃ onto said surface to form an antimicrobial surface layer having a thickness in the range of from 10 nm to 1.5µm, preferably using a thin film deposition technique.

## Patentansprüche

1. Medizinische Vorrichtung, die für den Kontakt mit lebendem Gewebe vorgesehen ist, wobei die medizinische Vorrichtung aus der Gruppe ausgewählt ist, die aus einem Zahnimplantat, einer im Knochen verankerten Hörvorrichtung und einem orthopädischen Implantat besteht, und ein Substrat mit einer antimikrobiellen Oberflächenschicht umfasst, die Ga₂O₃ enthält, **dadurch gekennzeichnet, dass**
diese Schicht nicht porös ist und eine Dicke im Bereich von 10 nm bis 1,5 µm hat und einen Galliumgehalt von mindestens 5 at% aufweist.

2. Medizinische Vorrichtung nach Anspruch 1, wobei das lebende Gewebe Weichgewebe ist.

3. Medizinische Vorrichtung nach einem der vorhergehenden Ansprüche, wobei die Schicht eine Dicke im Bereich von 10 nm bis 1 µm aufweist.

4. Medizinische Vorrichtung nach einem der vorhergehenden Ansprüche, wobei die Schicht einen Galliumgehalt von mindestens 10 at%, vorzugsweise von mindestens 20 at% aufweist.

5. Medizinische Vorrichtung nach einem der vorhergehenden Ansprüche, wobei die Schicht einen Galliumgehalt von bis zu 40 at% aufweist.

6. Medizinische Vorrichtung nach einem der vorhergehenden Ansprüche, wobei die Schicht eine homogene Schicht ist.

7. Medizinische Vorrichtung nach einem der vorhergehenden Ansprüche, wobei das Substrat ein metallisches Material, vorzugsweise Titan oder eine Titanlegierung, umfasst.

8. Medizinische Vorrichtung nach einem der Ansprüche 1 bis 6, wobei das Substrat ein keramisches Material umfasst.

9. Medizinische Vorrichtung nach einem der Ansprüche 1 bis 6, wobei das Substrat ein Verbundmaterial umfasst.

10. Medizinische Vorrichtung nach einem der vorhergehenden Ansprüche, wobei das Implantat ein Zahnimplantat ist.

11. Medizinische Vorrichtung nach Anspruch 10, wobei das Zahnimplantat ein Dentalaufbau ist.

12. Verfahren zur Herstellung einer medizinischen Vorrichtung nach einem der Ansprüche 1 bis 11, umfassend
a) Bereitstellen eines Substrats mit einer Oberfläche; und
b) Aufbringen von Ga₂O₃ auf die Oberfläche, um eine antimikrobielle Oberflächenschicht mit einer Dicke im Bereich von 10 nm bis 1,5 µm zu bilden, vorzugsweise unter Verwendung eines Dünnschichtabscheidungsverfahrens.

## Revendications

1. Dispositif médical destiné à venir en contact avec du tissu vivant, ledit dispositif médical étant sélectionné dans le groupe composé d'un implant dentaire, d'un dispositif d'audition ancré dans un os et d'un implant orthopédique et comprenant un substrat ayant une couche de surface antimicrobienne comprenant du Ga₂O₃,
**caractérisé en ce que**
ladite couche est non poreuse et a une épaisseur de l'ordre de 10 nm à 1,5 µm, et a une teneur en gallium d'au moins 5 at%.

2. Dispositif médical selon la revendication 1, dans lequel ledit tissu vivant est du tissu mou.

3. Dispositif médical selon l'une quelconque des revendications précédentes, dans lequel ladite couche a une épaisseur de l'ordre de 10 nm à 1 µm.

4. Dispositif médical selon l'une quelconque des revendications précédentes, dans lequel ladite couche a une teneur gallium d'au moins 10 at%, de préférence d'au moins 20 at%.

5. Dispositif médical selon l'une quelconque des revendications précédentes, dans lequel ladite couche à une teneur en gallium de jusqu'à 40 at%.

6. Dispositif médical selon l'une quelconque des revendications précédentes, dans lequel ladite couche est une couche homogène.

7. Dispositif médical selon l'une quelconque des revendications précédentes, dans lequel ledit substrat comprend un matériau métallique, de préférence du titane ou de l'alliage de titane.

8. Dispositif médical selon l'une quelconque des revendications 1 à 6, dans lequel ledit substrat comprend un matériau céramique.

9. Dispositif médical selon l'une quelconque des revendications 1 à 6, dans lequel ledit substrat comprend un matériau composite.

10. Dispositif médical selon l'une quelconque des revendications précédentes, dans lequel ledit implant est un implant dentaire.

11. Dispositif médical selon la revendication 10, dans lequel ledit implant est un pilier dentaire.

12. Procédé de production d'un dispositif médical selon l'une quelconque des revendications 1 à 11, comprenant
a) la prévision d'un substrat doté d'une surface ; et
b) l'application de Ga₂O₃ sur ladite surface pour former une surface antimicrobienne ayant une épaisseur de l'ordre de 10 nm à 1,5 µm, de préférence en utilisant une technique de dépôt de film mince.
